# EUROPEAN PATENT APPLICATION

(11) **EP 4 302 742 A1**
(43) Date of publication of application: **10.01.2024**
(21) Application number: 22305997.3
(22) Date of filing: 04.07.2022
(51) Int. Cl.: A61J 1/00, G01M 3/02, A61M 5/24, A61M 5/28, A61M 5/31, A61M 5/315

(54) **METHOD FOR DETERMINING A DISPLACEMENT OF A STOPPER OF A PRE-FILLED MEDICAL CONTAINER DURING THERMAL AND/OR PRESSURE VARIATIONS**

(71) Applicant: Becton Dickinson France, 38800 Le Pont de Claix (FR)
(72) Inventor: LEHEE, Guillaume, 38340 VOREPPE (FR); GIL, Ludovic, 38000 GRENOBLE (FR); PERRIN, Eloïse, 38320 EYBENS (FR)
(74) Representative: Regimbeau

(57) **Abstract**

The present disclosure relates to a method for determining a displacement of a stopper of a pre-filled medical container during thermal and/or pressure variations,
the pre-filled medical container comprising a barrel containing a medical composition, the stopper slidably arranged at a proximal end of the barrel and an air bubble extending from a free surface of the medical composition, the method comprising:
- calculating parameters for a thermomechanical model of the pre-filled medical container according to an analysis of the pre-filled medical container;
- based on the thermomechanical model of the pre-filled medical container, computing a thermal profile of the components;
- based on said computed thermal profiles of the components, computing the air pressure in the air bubble following a gas law and the displacement of the stopper over time during said thermal and/or pressure variations;
- based on said computed displacement, determining a risk of breaching the container closure integrity as a consequence of the cumulative proximal stopper displacement.

## Description

### TECHNICAL FIELD

The present disclosure relates to a method for determining a displacement of a stopper of a pre-filled medical container during thermal and/or pressure variations, and in particular, during at least one cycle. In particular said method may be used for determining a risk of loss of integrity of a pre-filled medical container due to plunger movement during a storage and/or shipment period all along the supply chain, including thermal and/or pressure cycle variations, and in particular, during at least one cycle. The disclosure also relates to a method for maintaining integrity of a pre-filled medical container during such a storage and/or shipment period.

### TECHNICAL BACKGROUND

Pre-filled medical containers are widely used in the pharmaceutical industry to provide medical users or patients with ready-to-use products to be injected into a patient's body.

During storage and/or shipment all along the supply chain, pre-filled medical containers may be subjected to at least one or a multiple and repeated combination of:
(i) one or more thermal cycle(s) comprising a large amplitude temperature decrease (e.g. from about 20°C to about -20°C or -80°C) and a large amplitude temperature increase (e.g. from about -20°C or -80°C to about 20°C);
(ii) one or more pressure cycle(s) comprising a large amplitude pressure decrease (e.g. from about 1000 hPa to about 500 hPA) and a large amplitude pressure increase (e.g. from about 500 hPA to about 1000 hPA);
(iii) one or more thermal fluctuation(s) comprising small amplitude temperature oscillations (e.g. +/- 10°C or +/-1°C around nominal temperature at 1 Hz or 1 mHz);
(iv) one or more pressure fluctuation(s) comprising small amplitude pressure oscillations (e.g. +/- 100hPa or +/-1hPa around nominal pressure at 1Hz or 1mHz).

Such temperature and/or pressure variations may for example be applied to pre-filled medical containers whose medical composition needs to be frozen to maintain its stability until use and/or shipped in reduced pressure transportation conditions (e.g. air transportation, truck in altitude).

Such thermal and/or pressure variations may generate loss of integrity of the medical container due to plunger movement in a non-sterile area. Container closure integrity (CCI) is defined as the ability of a medical container to prevent microbial ingress, entry of gases and/or debris, and loss of contents.

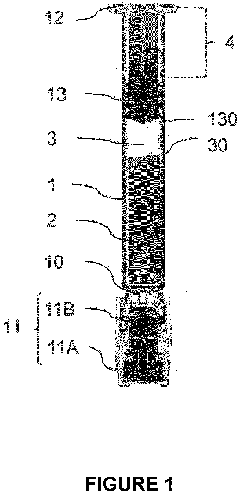

As shown in FIG. 1, a pre-filled medical container typically comprises a barrel 1 containing a medical composition 2.

The barrel 1 comprises a distal end comprising a tip 10 intended to expel the medical composition from the barrel, in particular to inject it into a patient's body. The tip 10 may comprise a needle (not shown) or may be configured to be connected to a needle.

The tip 10 may be sealingly closed by a tip cap 11. The tip cap may include a rigid cap 11A, typically made of plastic, and a resilient cap 11B, typically made of an elastomer.

The barrel 1 further comprises a proximal end 12 sealingly closed by a stopper 13. The stopper 13, which may be made of an elastomeric material, is slidable in the barrel 1 from a proximal rest position to a distal operative position for expelling the medical composition through the tip 10. The barrel 1 and/or the stopper 13 may be covered at least partially by a lubricant coating (not shown) to improve gliding of the stopper.

In the present text, the term "distal" refers to a portion of the pre-filled medical container that is farther from the user's hand (for example, on the side of the needle through which the medical composition may be injected into a patient's body) and the term "proximal" relates to a portion of the pre-filled medical container that is closer to the user's hand (for example, on the side of a flange of the barrel allowing the user to grip the pre-filled medical container).

The described pre-filled container is represented in a configuration where it is stored with its distal end down, i.e. oriented according to the direction of gravity.

At 20°C, the medical composition is typically in a liquid state and, in the illustrated orientation, an air bubble exists between the free (proximal) surface of the medical composition and the distal surface of the stopper (this space being commonly called "headspace").

The various types of materials involved in the prefilled medical container, especially the medical composition, the air trapped inside the barrel, and the elastomeric material of the stopper, generally have different behaviour when subjected to temperature variations as mentioned above.

During temperature changes, all the components will either contract or dilate based on their respective coefficient of thermal expansion. In addition, the medical composition will also change volume during its phase change (expansion during freezing, contraction during thawing).

Thus, the behavior of the prefilled medical container during thermal cycles is difficult to predict.

An effect of the above thermal phenomena is that the stopper moves in the barrel during the thermal cycle. During a temperature decrease, the shrinkage of air within the headspace may cause the stopper to move distally. However, freezing of the medical composition, which increases the volume of the medical composition, may cause the stopper to move proximally. During a temperature increase, the expansion of air within the headspace may cause the stopper to move proximally. However, thawing of the medical composition, which decreases the volume of the medical composition, may cause the stopper to move distally.

In addition, variation in pressure in the atmosphere surrounding the prefilled medical container may also contribute to the stopper movement inside the barrel.

During said back-and-forth movement of the stopper, the extreme positions reached by the stopper may vary.

In particular, the stopper may move in a region of the barrel that is not sterile. This region is located proximally from the proximal surface of the stopper when sterilization of the pre-filled medical container has been implemented. Movement of the stopper in said non-sterile region may breach integrity (in particular, sterility) of the medical composition.

It is thus desirable to avoid that the cumulative proximal displacement of the stopper from its initial sterilization position reaches a threshold distance, called sterile barrier height, in which the container closure integrity could be breached.

Currently, there does not exist any method allowing to estimate the risk of attaining the sterile barrier height, nor any method allowing to efficiently track the movement of the stopper for a batch of products.

One method comprises placing carbon black particles or similar markers on top of the stopper (in the non-sterile proximal region) after filling the barrel with the medical composition, and marking the initial position of the stopper. When the stopper moves, it may leave carbon black particles along the inner wall of the barrel and form a ring at its most proximal position. Determining the distance between the initial position of the stopper and the ring formed by the carbon black particles may allow estimating the amplitude of the proximal stopper movement. However, this method does not account for smaller back and forth displacements below the carbon black ring that cumulate and increase the risk of breaching the container closure integrity.

Another approach is to monitor the stopper movement with a camera. However, this method requires that a large volume be left around the medical container, which is not possible in conventional storage or shipment conditions, and it does not allow monitoring multiple medical containers at the same time. Thus, this method may be used in a laboratory environment but is not adapted to an industrial scale.

Documents WO 2015/138261 and US 2018/250474 propose mechanical solutions to prevent movement of the stopper during a thermal cycle. However, such solutions require a specific design of the medical container and may thus increase manufacturing complexity and cost.

### SUMMARY OF THE DISCLOSURE

A goal of the present disclosure is to determine with sufficient accuracy how the stopper is expected to move during pressure and/or temperature variations. Another goal of the present disclosure is to predict whether a pressure and/or thermal cycle or a plurality of pressure and/or thermal cycles and fluctuations are likely to cause a breach of the container closure integrity.

To that end, a first object of the disclosure is a method for determining a displacement of a stopper of a pre-filled medical container during thermal and/or pressure variations,
the pre-filled medical container comprising a barrel containing a medical composition, the stopper slidably arranged at a proximal end of the barrel and an air bubble extending from a free surface of the medical composition, the method comprising:
- calculating parameters for a thermomechanical model of the pre-filled medical container according to an analysis of the pre-filled medical container;
- based on the thermomechanical model of the pre-filled medical container, computing a thermal profile of the components;
- based on said computed thermal profiles of the components, computing the air pressure in the air bubble following a gas law and the displacement of the stopper over time during said thermal and/or pressure variations;
- based on said computed displacement, determining a risk of breaching the container closure integrity as a consequence of the cumulative proximal stopper displacement.

Preferably, said thermal and/or pressure variations may include at least one or a multiple and repeated combination of:
(i) one or more thermal cycle(s) comprising a large amplitude temperature decrease (e.g. from about 20°C to about -20°C or -80°C) and a large amplitude temperature increase (e.g. from about -20°C or -80°C to about 20°C);
(ii) one or more pressure cycle(s) comprising a large amplitude pressure decrease (e.g. from about 1000 hPa to about 500 hPA) and a large amplitude pressure increase (e.g. from about 500 hPA to about 1000 hPA);
(iii) one or more thermal fluctuation(s) comprising small amplitude temperature oscillations (e.g. +/- 10°C or +/-1°C around nominal temperature at 1 Hz or 1 mHz);
(iv) one or more pressure fluctuation(s) comprising small amplitude pressure oscillations (e.g. +/- 100hPa or +/-1hPa around nominal pressure at 1Hz or 1mHz).

In some embodiments, the thermomechanical model takes into account an average temperature of each component of the stopper during the at least one thermal variation and convection phenomena at interfaces between said elements during the at least one thermal variation to compute the displacement of the stopper over time.

In some embodiments, the thermomechanical model is a one-dimensional model assuming that the pre-filled medical container has an axisymmetric geometry.

In some embodiments, the thermomechanical model assumes that the medical composition freezes or thaws axisymmetrically during the at least one thermal variation.

In addition, the thermomechanical model may assume that the free surface of the medical composition remains flat during the thermal and/or pressure variations.

In some embodiments, the thermomechanical model takes into account a variable gliding force of the stopper depending on temperature.

In some embodiments, the resulting displacement is a displacement amplitude of the stopper during the at least one thermal variation.

In some embodiments, the resulting displacement of the stopper is a cumulative displacement of the stopper in proximal direction.

In some embodiments, the method further comprises drawing an abacus representing a maximum number of allowed thermal cycles as a function of a volume of the medical composition and a volume of air in the pre-filled medical container.

Another object of the present disclosure is a method for assessing a risk of loss of integrity of a pre-filled medical container during a storage and/or shipment period comprising at least one thermal and/or pressure variation, comprising:
- determining a sterile barrier height defined as being a distance between the most distal and the most proximal points of contact of the sealing stopper with the barrel in an initial position of the stopper;
- determining a resulting displacement of the stopper during said storage and/or shipment period by the method as described above;
- comparing said resulting displacement with the sterile barrier height;
- determining that a risk of loss of integrity exists if the resulting displacement is greater than the sterile barrier height, or determining that no risk of loss of integrity exists if the resulting displacement is smaller than the sterile barrier height.

In some embodiments, the method further comprises determining a maximum number of thermal and/or pressure variations allowing maintaining integrity of the pre-filled medical container so that the resulting displacement of the stopper for said maximum number of thermal and/or pressure variations is lower than the sterile barrier height.

Said maximum number of thermal cycles may be computed from a ratio between the sterile barrier height and the resulting displacement of the stopper during a single thermal cycle.

The present disclosure further relates to a method for maintaining integrity of a pre-filled medical container during a storage and/or shipment period including at least one thermal and/or pressure variation, the pre-filled medical container comprising a barrel containing a medical composition and a stopper in gliding engagement within the barrel, comprising:
- determining a maximum number of thermal and/or pressure variations with the method as described above;
- subjecting the pre-filled medical container to a number of thermal and/or pressure variations smaller than said determined maximum number.

In some embodiments, during each thermal variation the temperature decreases to - 80°C or below -80°C.

### BRIEF DESCRIPTION OF THE FIGURES

Further features and advantages of the invention will appear in the following description, based on the appended drawings, wherein:
- FIG. 1 is a sectional view of a pre-filled medical container;
- FIG. 2 is a representation of a thermomechanical model of the pre-filled medical container of FIG.1;
- FIGS. 3A and 3B represent the main interfaces taken into account in the model, respectively when the medical composition is in a liquid state and when the medical composition is partially frozen during a temperature decrease;
- FIG. 4 is an example of a simulated displacement (in mm) of the stopper over time (in s), assuming that the stopper is free to glide within the barrel;
- FIG. 5 is an example of a simulated displacement (in mm) of the stopper over time (in s), taking into account a variable gliding force depending on the temperature;
- FIG. 6 represents how the stopper displacement amplitude and the cumulated positive stopper displacement can be determined from a curve representing the simulated displacement of the stopper over time;
- FIG. 7 is an example of an abacus representing a mapping of the stopper displacement amplitude and the maximum number of thermal cycles depending on the volume of the medical composition and the size of the headspace;
- FIG. 8 is an example of an abacus representing a mapping of the cumulated positive stopper displacement and the maximum number of thermal cycles depending on the volume of the medical composition and the size of the headspace.

### DETAILED DESCRIPTION OF EMBODIMENTS

The present disclosure is based on the definition of a simplified thermomechanical model of a pre-filled medical container accounting for a plurality of parameters relating to the design of the pre-filled medical container and the conditions of the thermal / pressure variations to which the pre-filled medical container will be subjected.

In particular, said thermal and/or pressure variations may include one or a multiple and repeated combination of:
- one or more thermal cycle(s) comprising a large amplitude temperature decrease (e.g. from about 20°C to about -20°C or -80°C) and a large amplitude temperature increase (e.g. from about -20°C or -80°C to about20°C);
- one or more pressure cycle(s) comprising a large amplitude pressure decrease (e.g. from about 1000hPa to about 500hPA) and a large amplitude pressure increase (e.g. from about 500hPA to about 1000hPA);
- one or more thermal fluctuation(s) comprising small amplitude temperature oscillations (e.g. +/- 10°C or +/-1°C around nominal temperature at 1Hz or 1mHz);
- one or more pressure fluctuation(s) comprising small amplitude pressure oscillations (e.g. +/- 100hPa or +/-1hPa around nominal pressure at 1Hz or 1mHz).

The pre-filled medical container has already been described with reference to FIG. 1.

In FIG. 1, the pre-filled container is represented in a configuration where it is stored with its distal end down, i.e. oriented according to the direction of gravity.

However, in other embodiments, the pre-filled container may be oriented with its distal end up, i.e. in a direction opposite to the direction of gravity, or perpendicular to the direction of gravity, or with any other angle relative to the direction of gravity.

The thermomechanical model is advantageously defined to take into account at least the following parameters of the pre-filled medical container and the conditions of the thermal / pressure cycle:
- range of temperature and pressure attained over the thermal cycle; for example, said temperature may attain -80°C or even below -80°C;
- geometrical characteristics of the barrel, such as the inner and outer diameters of the barrel;
- design of the stopper, in particular the sterile barrier height;
- volume of the medical composition in the barrel;
- size of the headspace (i.e. the air bubble between the medical composition and the stopper, when the pre-filled medical container is positioned with its distal end oriented towards the ground, according to the direction of the gravity).

The sterile barrier height is defined as the distance between the most proximal and the most distal points of contact of the stopper with the barrel. Said distance can be smaller than the height of the stopper since the stopper may not be in contact with the barrel over its whole height. For example, if the stopper is in contact with the barrel via distal and proximal ribs, the sterile barrier height is the distance between said ribs.

The thermomechanical model may be simplified based on assumptions on the geometry of the medical container, for example a cylindric axisymmetric geometry of the barrel and the stopper. The model can thus be a 1D (one dimension) model.

The thermomechanical system may also be based on the assumption that the temperature is uniform in each component of the medical container; said temperature can be chosen as an average temperature of the component (of course, this temperature varies over the thermal cycle).

In the thermomechanical model, the medical composition may be considered as being water, which is usually the main constituent of the medical composition, or any other appropriate fluid whose thermal and mechanical properties (e.g. freezing temperature, fusion enthalpy, conduction, convection, coefficient of thermal expansion, specific heat) are known. In the following description, the fluid is considered to be water, only for sake of illustration and not limitation.

Depending on the stage of the thermal cycle, said water may be in a liquid state or at least partially frozen (ice).

If the medical container is stored or shipped with the distal tip down (according to the direction of gravity), as illustrated in FIG. 1, the thermomechanical model may be based on the assumption that the freezing and thawing of the water occur according to an axisymmetric geometry. In particular, it is assumed that the water freezes first at the interface with the barrel (i.e. with the inner lateral and distal surfaces of the barrel) and at the interface with the air bubble (i.e. at the free surface of the medical composition). It is also assumed that the ice melts first at the same interfaces. In addition, it may be assumed that the free (proximal) surface of the medical composition remains flat even when the composition freezes.

If the medical container is stored or shipped with another orientation relative to the direction of gravity, the skilled person will be able to adjust the thermomechanical model in order to take into account the behavior of the components in this orientation. In this adjustment, account will also be paid to the fact that, if the medical container is stored or shipped with the distal tip up (opposite position to the position illustrated in FIG. 1), the air bubble is located between the composition and the distal tip of the medical container. More generally, the air bubble always extends above the free surface of the medical composition.

Despite the above assumptions that are used to simplify the model and decrease the computation time, the thermomechanical model may take into account physical and thermodynamical features of the pre-filled medical container in order to provide a sufficiently accurate simulation of the behavior of the stopper.

In particular, the thermomechanical model takes into account the thermal expansion of each component of the medical container and its change in specific heat.

Advantageously but optionally, the thermomechanical model may take into account the gliding performance of the stopper, for example the activation and gliding forces at various temperatures of the thermal cycle. The activation force is the force required to initiate the movement of the stopper at the beginning of the injection of the composition; the gliding force is the force required to move the stopper within the barrel during the injection.

It is to be noted that a complete model of the pre-filled medical container could be computed using finite element method, which requires meshing the whole barrel. Such a computation requires a few hours to calculate the evolution of the temperature of the medical composition and of the temperature and pressure of the air bubble, the stopper being considered to be fixed relative to the barrel. However, such a simulation would closely depend on the temperature / pressure profile during the thermal cycle, the design of the stopper, the volume of medical composition and air within the barrel. Thus, in practice, it would be a huge burden for the skilled person to implement such a complete model to determine a risk of breach of closure integrity for various medical containers and/or various conditions of thermal cycles.

As a comparison, a thermomechanical model according to the present disclosure can be run quickly (in a few seconds) to estimate the displacement of the stopper during one or several thermal and/or pressure cycle(s).

Said thermomechanical model thus allows easily simulating the risk of breach of integrity for a plurality of designs of pre-filled medical containers and a plurality of thermal / pressure cycles.

In particular, as will be explained in more details below, the thermomechanical model may allow determining abacuses allowing the skilled person to identifying the risk of integrity breach (expressed as a maximum number of cycles allowing preserving closure integrity) for a given design of the pre-filled medical container and given conditions of the thermal / pressure cycles. For example, said abacuses may represent, for a specific design of the pre-filled medical container, the maximum number of cycles as a function of the volume of the medical composition and the volume of the headspace.

The thermomechanical model can be translated into an algorithm that is configured to, based on input data relating to the pre-filled medical container and the thermal and/or pressure cycle, compute the temperatures of the different components, the pressure in the headspace and the displacement of the stopper and provide as an output the maximum number of thermal cycles allowing preserving closure integrity. Such an algorithm may in particular be programmed using Scilab programming language, or any other programming language, and be implemented on a common use computer, such as a lap-top.

In practice, a user interface may be provided, allowing a user to enter specific parameters of the pre-filled medical container, such as:
- geometrical characteristics of the barrel, such as the inner and outer diameters of the barrel;
- the nominal volume of the medical composition;
- the nominal height of the air bubble in the headspace;
- the initial temperature and pressure;
- the minimum temperature attained during the thermal cycle (e.g. cold storage temperature);
- the cold storage duration;
- the thaw duration, i.e. the time required to bring the medical composition from a frozen state to a liquid state;
- the size of the stopper;
- the activation and gliding forces of the stopper at initial and cold storage temperature.

Based on the convection models at the various interfaces, the computer may calculate the temperature and behavior of the components of the pre-filled medical container at a plurality of times, assuming that the evolution of the parameters of the pre-filled medical container is quasi-static. In particular, the displacement of the stopper relative to its initial position may be computed at each of said plurality of times, based on the behavior of the solid components, the freezing or thawing of the medical composition, and the variation of pressure of the air bubble and the outside atmosphere.

The user interface may be adapted to display, as an output, a curve showing the displacement of the stopper over time, a maximum number of cycles allowed to maintain closure integrity, and/or an abacus representing the maximum number of cycles for various parameters of the pre-filled medical container.

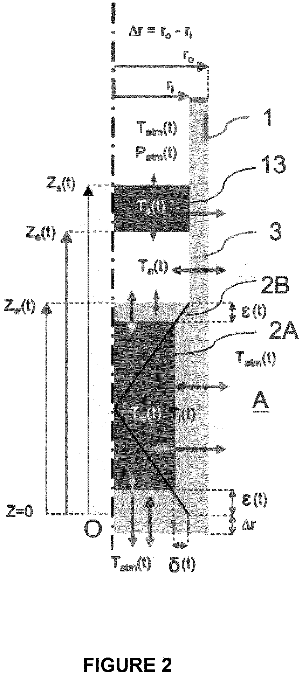

FIG. 2 illustrates a representation of the simplified model with the interactions between the components of the pre-filled medical container illustrated in FIG. 1.

The model is defined in a coordinate system comprising a radial direction r (which is the horizontal direction in FIG. 2) and a longitudinal direction Z (which is the vertical direction in FIG. 2). The origin O of the coordinate system is located at the center of the inner surface of the distal end of the barrel 1. The barrel is modeled as a cylinder having a circular base with a closed distal end and an open proximal end, with a constant thickness. The tip of the barrel and the tip cap have thus not been modeled in the illustrated example. The thickness Δr of the barrel is computed as the difference between the outer diameter rₒ and the inner diameter rᵢ of the barrel.

In the illustrated model, it is considered that the medical composition comprises a liquid portion 2A and a frozen (solid ice) portion 2B. As explained above, the medical composition may be assimilated to water or any other suitable fluid whose thermal and mechanical properties are known.

In the illustrated model, ε₁ defines the thickness of the frozen portion of the liquid 2B on the distal end, ε₂ define the thickness of the frozen portion of the liquid 2B on the proximal end (ε₁ and ε₂ may be equal), and δ define the thickness of the frozen portion of liquid 2B on the radial direction. These thicknesses of the frozen portion 2B in their respective directions are assumed to be constant over the length of their respective directions. However, the thicknesses ε₁, ε₂ and δ vary over time, which is expressed by ε₁(t), ε₂(t) and δ(t). The liquid portion 2A and the frozen portion 2B have a respective temperature T_{w}(t) and Tᵢ(t) which varies over time.

The same methodology is used to model the thawing step when the frozen liquid melts.

In the headspace 3, the air has a temperature Tₐ(t) which varies over time and a pressure that varies according to a gas law. In preferred embodiment, the ideal gas law is used.

The stopper 13 has a temperature Tₛ(t) which varies over time.

The outside atmosphere A surrounding the pre-filled medical container is also taken into account. The outside atmosphere has a temperature Tₐₜₘ(t) varying over time and a pressure Pₐₜₘ(t) varying over time.

The thermal exchanges with the environment and between the components are represented by the arrows.

The level of the medical composition in the barrel is represented by the distance Z_{w}(t) from the origin O. The position of the stopper is represented by the distance Zₐ(t) of its distal surface from the origin O and the distance Zₛ(t) of its proximal surface from the origin O.

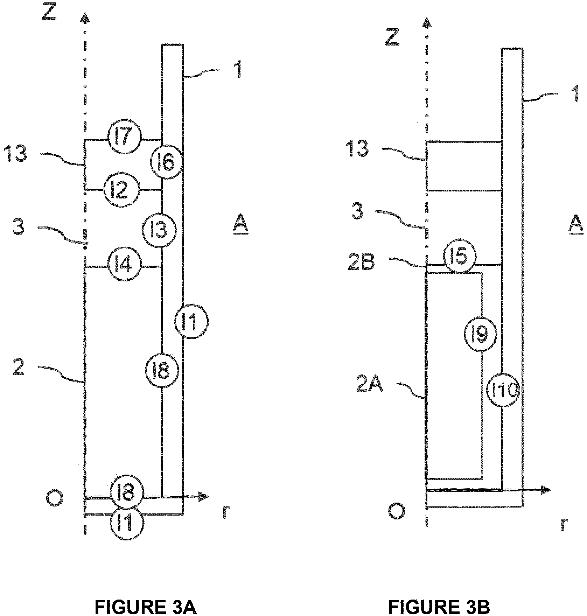

FIGS. 3A and 3B represent the main interfaces within the pre-filled medical container and between the pre-filled medical container and the outside atmosphere.

The following table describes the interfaces and the thermal model used at each interface.

| Reference | Interface | Model |
|---|---|---|
| 11 | Outside atmosphere / glass (barrel) | Convection |
| 12 | Air (headspace) / rubber (stopper) | Convection |
| 13 | Air (headspace) / glass (barrel) | Convection |
| 14 | Air (headspace) / liquid water (medical composition) | Convection |
| 15 | Air (headspace) / ice (medical composition) | Convection |
| 16 | Rubber (stopper) / glass (barrel) | Continuous temperature |
| 17 | Rubber (stopper) / air (outside atmosphere) | Convection |
| 18 | Liquid water (medical composition) / glass (barrel) | Convection |
| 19 | Liquid water / ice | Convection |
| 110 | Ice (medical composition) / glass (barrel) | Continuous temperature |

The skilled person is able to determine the convection models between the components based on the properties of the materials, that are accessible for example in databases of materials.

If appropriate, the skilled person may validate or calibrate the thermomechanical model using a few complete simulations of the pre-filled medical container, by comparing the results obtained by the thermomechanical model and the complete finite element model in a few specific configurations. In addition, the skilled person may validate or calibrate the thermomechanical model using experimental data measured on a few specific configurations of the pre-filled medical container, by comparing the results obtained by the thermomechanical model and the measured results in said configurations. However, once the thermomechanical model has been validated, such complete simulations or experimental measurement are not necessary to run simulations.

In a worst case simulation, the stopper may be considered to be free to glide within the barrel (otherwise said, no frictional force opposes to the movement of the stopper).

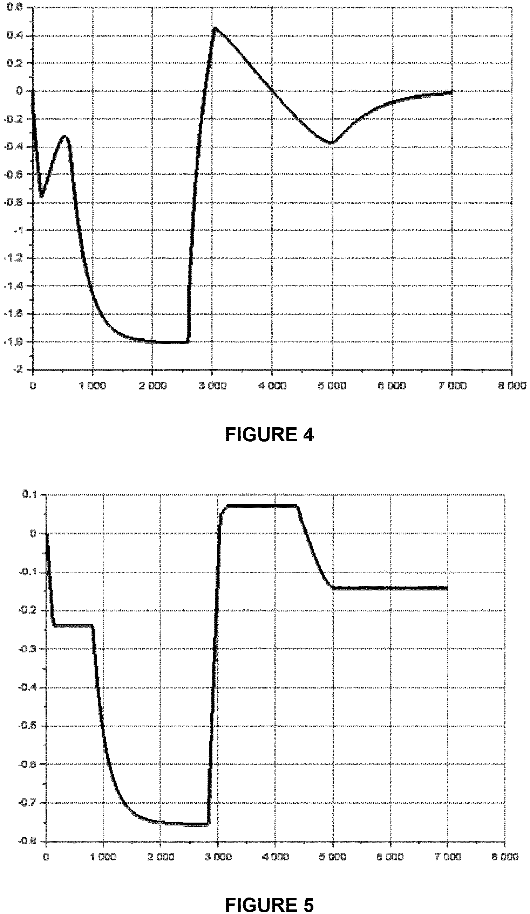

FIG. 4 is an example of a simulated displacement of the stopper over time, assuming that the stopper is free to glide within the barrel.

However, a more realistic simulation may account for an activation force and a gliding force of the stopper. Advantageously, said activation and gliding forces depend on the temperature and on the velocity of the stopper. The activation force and the gliding force may be determined based on the material of the stopper, the barrel and, if any, a lubricant coating deposited onto the barrel and/or the stopper. If appropriate, experimental tests can be carried out to measure the activation force and the gliding force at different temperatures, and the measurements can be used to calibrate the activation force and the gliding force in the model.

For example, the activation force, which is the force required to begin moving the stopper from a fixed position, could be estimated to be about 0.1 N at 20°C and about 1N at -80°C.

FIG. 5 is an example of a simulated displacement of the stopper over time, for the same design of the pre-filled medical container as in FIG. 4, assuming that the activation and the gliding force are equal to of 0.1 N at 20°C and 1N at -80°C.

It can be seen that the activation and gliding forces have an effect on the shape and the amplitude of the displacement curve, the gliding force significantly reduces the amplitude of displacement of the stopper.

Based on the displacement curves of FIG. 4 or FIG. 5, it is possible to determine a resulting displacement of the stopper, which can be the amplitude of displacement of the stopper and/or the cumulated positive displacement of the stopper.

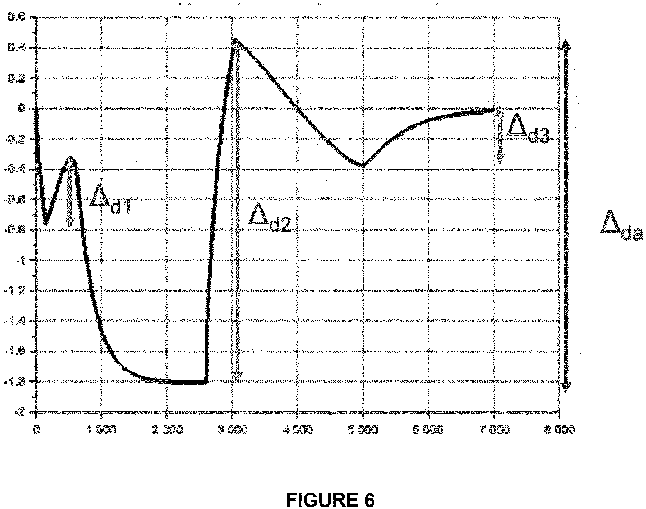

For example, as shown in FIG. 6 which is based on the curve of FIG. 4, the amplitude of displacement Δ_{da} of the stopper is the difference between the most proximal and the most distal position of the stopper.

Still referring to FIG. 6, the cumulated proximal displacement Δ_{cd} of the stopper is the sum of the displacements of the stopper in the proximal direction. In the example of FIG. 6, the thermal cycle involves three displacements of the stopper in the proximal direction, respectively Δ_{d1}, Δ_{d2} and Δ_{d3}. The cumulated proximal displacement Δ_{cd} is the sum of Δ_{d1}, Δ_{d2} and Δ_{d3}.

The resulting displacement of the stopper can thus be compared to the sterile barrier height:
- if the resulting displacement of the stopper is greater than the sterile barrier height, there is a risk of loss of container closure integrity;
- if the resulting displacement of the stopper is smaller than the sterile barrier height, there is no risk of loss of container closure integrity.

Due to friction, the position of the stopper at the end of a cycle is not necessarily the same as the initial position. Therefore the displacements are not the same in all the cycles.

The maximum number of thermal cycles can be computed by summing the cumulated proximal displacements Δ_{cd} of each thermal and/or pressure variation until it becomes greater than the sterile barrier height.

A similar computation of the resulting displacement (displacement amplitude and/or cumulative positive displacement) can be made based on a curve as shown in FIG. 5, taking into account the gliding force of the stopper.

Of course, the shape and amplitude of the curves of FIGS. 4 and 5 are linked to a specific design of the pre-filled medical container and are provided only as an illustrative example. For different designs of pre-filled medical containers, or different filling conditions (volume of the medical composition and of the headspace), the shape and amplitude of these curves may vary, and the resulting displacement of the stopper may also vary.

These simulations may advantageously be used to compute abacuses allowing the skilled person to easily determine a level of risk of breaching integrity of the pre-filled medical container, or determine a maximum number of thermal cycles to avoid breaching said integrity.

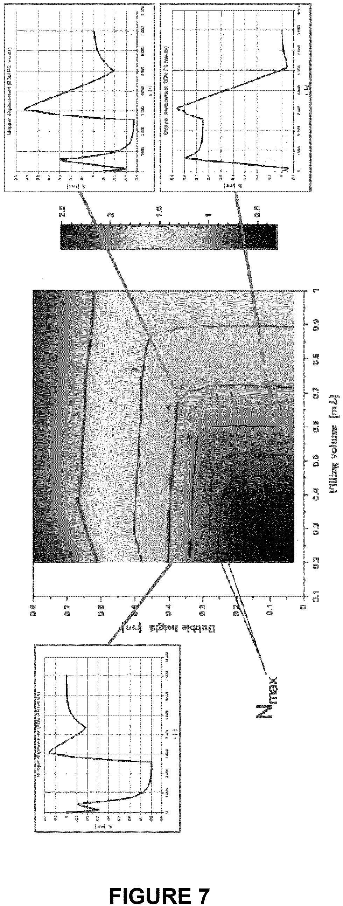

FIG. 7 illustrates an abacus drawn from a plurality of curves of the type shown in FIG. 4 (if no gliding force is taken into account) or FIG. 5 (if the gliding force is taken into account) for a plurality of filling conditions.

The abacus of FIG. 7 represents the displacement amplitude Δ_{da} as a function of the volume of the medical composition in mL (in abscissa) and the height of the headspace in cm (in ordinate). The value of the displacement amplitude is associated to a color map ranging from 0 to 2.5 mm.

Three curves of the stopper displacement over time for different values of the volume of the medical composition and the height of the headspace (all the other parameters of the pre-filled medical container being identical for all simulations) used for drawing the abacus have been represented. The displacement amplitude has been measured on each of said curves, as shown in FIG. 6, and used to plot a point on the abacus. Values of the displacement amplitude between adjacent points may be estimated by an interpolation. In these simulations, no gliding force has been taken into account but, as noted above, a similar abacus could have been drawing using curves obtained by simulations taking into account the gliding force.

In addition to the color map of the displacement amplitude, the abacus also displays a plurality of curves, each representing a maximum number Nₘₐₓ of thermal cycles allowing not breaching integrity of the medical container. As explained previously, said maximum number of thermal cycles is computed as being the floor function of the ratio between the sterile barrier height and the displacement amplitude Δ_{da}. In the illustrated example, the sterile barrier height is set to 5.5 mm.

In practice, the skilled person can use such an abacus to determine a suitable couple of values for the volume of the medical composition and the height of the headspace in order to ensure a given number of allowed thermal cycles. For example, if the goal is to guarantee closure integrity for at least five thermal cycles, the skilled person may select the volume of the medical composition and the height of the headspace in the region of the abacus located below the curve corresponding to Nₘₐₓ = 5.

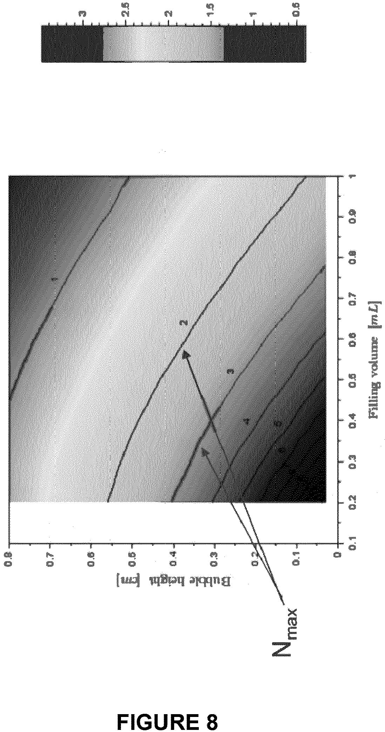

Alternatively, the abacus may represent the cumulative stopper displacement Δ_{cd} as a function of the volume of the medical composition in mL (in abscissa) and the height of the headspace in cm (in ordinate), as shown in FIG. 8. The value of the cumulative displacement is associated to a color map ranging from 0.5 to 3.5 mm.

Although not represented in FIG. 8, the abacus may be drawn in a similar way as the abacus of FIG. 7, using a plurality of curves of the stopper displacement over time for different values of the volume of the medical composition and the height of the headspace (all the other parameters of the pre-filled medical container being identical for all simulations). The cumulative displacement has been measured on each of said curves, as illustrated in FIG. 6, and used to plot a point on the abacus. Values of the cumulative displacement between adjacent points may be estimated by an interpolation. In these simulations, no gliding force has been taken into account but, as noted above, a similar abacus could have been drawing using curves obtained by simulations taking into account the gliding force.

In addition to the color map of the cumulative displacement, the abacus also displays a plurality of curves, each representing a maximum number Nₘₐₓ of thermal cycles allowing not breaching integrity of the medical container. In this particular case, said maximum number of thermal cycles is computed as being the floor function of the ratio between the sterile barrier height and the displacement amplitude Δ_{cd}. In the illustrated example, the sterile barrier height is set to 5.5 mm.

In practice, the skilled person can use such an abacus to determine a suitable couple of values for the volume of the medical composition and the height of the headspace in order to ensure a given number of allowed thermal cycles. For example, if the goal is to guarantee closure integrity for at least five thermal cycles, the skilled person may select the volume of the medical composition and the height of the headspace in the region of the abacus located below the curve corresponding to Nₘₐₓ = 5.

Of course, the abacuses are only provided as illustrative examples and may vary depending on the design of the pre-filled medical container and on the conditions of the thermal / pressure cycles.

It is to be noted that the thermal and/or pressure cycles may not be applied consecutively. For example, the pre-filled medical container may be subjected to two separate thermal and/or pressure cycles, each corresponding to a shipment from one location to another one.

## Claims

1. Method for determining a displacement of a stopper of a pre-filled medical container during thermal and/or pressure variations,
the pre-filled medical container comprising a barrel containing a medical composition, the stopper slidably arranged at a proximal end of the barrel and an air bubble extending from a free surface of the medical composition, the method comprising:
- calculating parameters for a thermomechanical model of the pre-filled medical container according to an analysis of the pre-filled medical container;
- based on the thermomechanical model of the pre-filled medical container, computing a thermal profile of the components;
- based on said computed thermal profiles of the components, computing the air pressure in the air bubble following a gas law and the displacement of the stopper over time during said thermal and/or pressure variations;
- based on said computed displacement, determining a risk of breaching the container closure integrity as a consequence of the cumulative proximal stopper displacement.

2. Method according to claim 1, wherein the thermomechanical model takes into account an average temperature of each component of the stopper during the at least one thermal variation and convection phenomena at interfaces between said elements during the at least one thermal variation to compute the displacement of the stopper over time.

3. Method according to claim 1 or claim 2, wherein the thermomechanical model is a one-dimensional model assuming that the pre-filled medical container has an axisymmetric geometry.

4. Method according to any one of claims 1 to 3, wherein the thermomechanical model assumes that the medical composition freezes or thaws axisymmetrically during the at least one thermal variation.

5. Method according to claim 4, wherein the thermomechanical model assumes that the free surface of the medical composition remains flat during the thermal and/or pressure variations.

6. Method according to any one of claims 1 to 5, wherein the thermomechanical model takes into account a variable gliding force of the stopper depending on temperature.

7. Method according to any one of claims 1 to 6, wherein the resulting displacement is a displacement amplitude of the stopper during the at least one thermal variation.

8. Method according to any one of claims 1 to 6, wherein the resulting displacement of the stopper is a cumulative displacement of the stopper in proximal direction.

9. Method according to any one of claims 1 to 8, further comprising drawing an abacus representing a maximum number (Nₘₐₓ) of allowed thermal cycles as a function of a volume of the medical composition and a volume of air in the pre-filled medical container.

10. Method for assessing a risk of loss of integrity of a pre-filled medical container during a storage and/or shipment period comprising at least one thermal and/or pressure variation, comprising:
- determining a sterile barrier height defined as being a distance between the most distal and the most proximal points of contact of the sealing stopper with the barrel in an initial position of the stopper;
- determining a resulting displacement of the stopper during said storage and/or shipment period by the method as claimed in claims 1 to 9;
- comparing said resulting displacement with the sterile barrier height;
- determining that a risk of loss of integrity exists if the resulting displacement is greater than the sterile barrier height, or determining that no risk of loss of integrity exists if the resulting displacement is smaller than the sterile barrier height.

11. Method according to claim 10, further comprising determining a maximum number of thermal and/or pressure variations allowing maintaining integrity of the pre-filled medical container so that the resulting displacement of the stopper for said maximum number of thermal and/or pressure variations is lower than the sterile barrier height.

12. Method according to claim 11, wherein said maximum number of thermal variations is computed from a ratio between the sterile barrier height and the resulting displacement of the stopper during a single thermal cycle.

13. Method for maintaining integrity of a pre-filled medical container during a storage and/or shipment period including at least one thermal and/or pressure variation, the pre-filled medical container comprising a barrel containing a medical composition and a stopper in gliding engagement within the barrel, comprising:
- determining a maximum number of thermal and/or pressure variations with the method according to any one of claims 11 or 12;
- subjecting the pre-filled medical container to a number of thermal and/or pressure variations smaller than said determined maximum number.

14. Method according to claim 12, wherein during each thermal variation the temperature decreases to -80°C or below -80°C.
